# EUROPÄISCHE PATENTSCHRIFT

(11) **EP 1 648 308 B1**
(45) Veröffentlichungstag und Bekanntmachung des Hinweises auf die Patenterteilung: **10.11.2010**
(21) Anmeldenummer: 04763571.9
(22) Anmeldetag: 28.07.2004
(51) Int. Cl.: A61B 17/00

(54) **VERSCHLUSSSTOPFEN FÜR EINE ÖFFNUNG IN EINER WAND EINES GEFÄSSES ODER HOHLORGANS**
SEALING CLOSURE FOR AN OPENING IN A WALL OF A VESSEL OR HOLLOW ORGAN
BOUCHON DE FERMETURE POUR OUVERTURE PRATIQUEE DANS UNE PAROI DE VAISSEAU OU D'ORGANE CREUX

(30) Priorität: 30.07.2003 DE 10335648
(43) Veröffentlichungstag der Anmeldung: 26.04.2006
(73) Patentinhaber: Eberhard-Karls-Universität Tübingen Universitätsklinikum, 72076 Tübingen (DE)
(72) Erfinder: WENDEL, Hans-Peter, 72336 Balingen (DE); NEUMANN, Bernd, 72108 Rottenburg-Wurmlingen (DE)
(74) Vertreter: Findeisen, Marco
(86) Internationale Anmeldenummer: PCT/EP2004/008458
(87) Internationale Veröffentlichungsnummer: WO 2005/011352

(56) Entgegenhaltungen:
- EP-A- 1 000 958
- WO-A-99/38454
- DE-C- 19 626 397
- US-A1- 2002 173 810
- US-A1- 2003 055 198

## Beschreibung

Die vorliegende Erfindung betrifft einen Verschlussstopfen für eine Öffnung in einer Wand eines Gefäßes oder Hohlorgans eines tierischen oder menschlichen Körpers, insbesondere eines Blutgefäßes.

Die vorliegende Erfindung betrifft allgemein Vorrichtungen, um Öffnungen in Wänden von Gefäßen oder Hohlorganen perkutan zu verschließen. Insbesondere betrifft die vorliegende Erfindung das perkutane Verschließen von arteriellen oder venösen Punktionsstellen, die üblicherweise nur durch die Haut zugänglich sind.

Viele diagnostische und interventionelle vaskuläre Verfahren werden transluminal durchgeführt, wozu an einer geeigneten Zugangsstelle ein Katheter in das vaskuläre System eingeführt und durch das vaskuläre System zu einer Zielregion geführt wird, wozu etablierte Techniken verwendet werden. Dieser vaskuläre Zugang wird beispielsweise durch die gut bekannte Seldinger-Technik geschaffen.

Wenn der vaskuläre Zugang nicht länger benötigt wird, muss das eingeführte Schleusenröhrchen entfernt und die Blutung an der Punktionsstelle gestoppt werden. Das Schleusenröhrchen wird in der Fachwelt auch als Schleuse, Zugangsschleuse oder Introducer bezeichnet.

Ein perkutaner vaskulärer Zugang wird beispielsweise zur Durchführung von perkutanen transluminalen angiologischen Interventionen benötigt, bei denen das Schleusenröhrchen, das in der Regel einen Durchmesser von 5 - 8 French entsprechend 1,7 - 2,7 mm hat, meist in die Arteria femoralis eingeführt wird. Zu den mit einem derartigen Zugang durchgeführten Verfahren zählen beispielsweise die Angiographie, Angioplastie, Laserablation, Stentplatzierung, intravaskuläre Bildaufnahme, perkutane transluminale koronare Angioplastie etc. Es sind aber auch andere Verfahren bekannt, bei denen ein perkutaner Zugang zu Hohlorganen und Lumen im Körper benötigt wird, wie beispielsweise laparoskopische Verfahren, endoskopische Verfahren und dergleichen.

Um Blutungskomplikationen zu vermeiden und den Patienten zu einer schnellen Mobilität zu verhelfen, ist es wichtig, die Blutung aus dem beispielsweise arteriellen Gefäß nach dem Ziehen des Schleusenröhrchens schnell zu stoppen. Dies bereitet insbesondere bei Patienten Probleme, die Antikoagulantien, Thrombozyteninhibitoren oder eine Lysetherapie erhalten haben.

Ein gängiges Verfahren zum Stoppen der Blutung besteht darin, die Punktionsstelle bis zum Ende der Blutung mittels manueller Kompression und anschließendem Druckverband zu versorgen. Nachteilig ist hierbei, dass dieses Verfahren sehr zeitaufwendig ist und häufig eine manuelle Kompression von bis zu einer Stunde erfordert, bevor die Hämostase sichergestellt ist. Ein weiteres Problem bei diesem Verfahren besteht darin, dass es für den Patienten wegen der Anwendung von exzessivem Druck unangenehm ist und darüber hinaus das entsprechende Blutgefäß völlig verschließen kann, was zu Ischämien und/oder Thrombosen führen kann.

Um diese Probleme zu beseitigen, beschreibt die US 5,417,699 einen Naht-Automaten mit einem langen Röhrchen, an dessen distalem Ende Nadeln und entsprechendes Nahtmaterial angeordnet sind. Der Naht-Automat wird in das noch gelegte Schleusenröhrchen eingeführt, bis sein distales Ende im Lumen des zu verschließenden Gefäßes zu liegen kommt. Dann wird das Schleusenröhrchen so weit zurückgezogen, dass es aus der zu verschließenden Öffnung in der Wand freikommt und das distale Ende des Naht-Automaten freigibt, woraufhin die Nadeln sich aufspreizen. Die Nadeln werden jetzt durch die die zu verschlieβende Öffnung umgebende Wand nach außen gestoßen, dort an ihrer Spitze erfasst und wieder in das Schleusenröhrchen hineingezogen. Beim Hineinziehen des Naht-Automaten in das Schleusenröhrchen wird das am distalen Ende der Nadeln befestigte Nahtmaterial jetzt durch die Wand gezogen und kann anschließend verknotet werden.

Der von der Firma Perclose Inc., Sunnyvale, Kalifornien unter dem Produktnamen Prostar vertriebene Naht-Automat ist technisch sehr kompliziert und erfordert große Erfahrung bei dem Bedienungspersonal. Darüber hinaus ist dieses System teuer und aufwendig in der Anwendung.

Die älteste Vorrichtung zum perkutanen Verschluss von Öffnungen in Blutgefäßen wird unter dem Produktnamen *VasoSeal^{®}* als *vascular hemostatic device* von Datascope Corporation, Montvale, N.J., USA vertrieben. Das Verfahren beruht primär auf Kollagen-induzierter Thrombusgenerierung. Zu diesem Zweck wird ein gereinigter Kollagenstopfen eingeführt, der die Bildung einer hämostatischen Kappe direkt oberhalb der Punktionsstelle induziert.

Um den Kollagenstopfen an Ort und Stelle zu bringen, muss zuerst das gelegte Schleusenröhrchen gezogen und durch ein Röhrchen mit relativ großem Durchmesser ersetzt werden, durch das dann der Kollagenstopfen platziert wird. Zum Erzielen der Hämostase ist es jedoch häufig erforderlich, für einen bestimmten Zeitraum eine manuelle Kompression durchzuführen, so dass auch hier die damit im Zusammenhang stehenden, oben bereits diskutierten Probleme auftreten können.

Ein *Angio-Seal*^{®} genanntes Verfahren beruht auf mechanischen Kräften, die nach der Sandwichtechnik aufgebracht werden, sowie auf Kollagen-induzierter Thrombusbildung. Durch einen Anker innerhalb der Arterie, der den Kollagenpfropfen in dem Gewebe führt und hält, wird die Punktionsstelle mechanisch blockiert. Auch bei diesem Verfahren muss zunächst das bestehende Schleusenröhrchen entfernt werden, bevor ein neues Schleusenröhrchen gelegt werden kann, durch das dann der Kollagenstopfen an Ort und Stelle gebracht wird. Der Kollagenstopfen wird dann über einen Stampfer gegen die Außenwand der Arterie gedrückt, wobei der Stampfer über eine Feder auf den Kollagenstopfen gedrückt wird, die gleichzeitig den Anker von innen über ein Nahtmaterial gegen die Öffnung zieht.

Bei den beiden zuletzt beschriebenen Verfahren ist darüber hinaus von Nachteil, dass zunächst das bestehende Schleusenröhrchen gegen ein neues Röhrchen ausgetauscht werden muss, was insbesondere bei stark blutenden Öffnungen zu großen Problemen führen kann. Darüber hinaus ist mit den erforderlichen Manipulationen ein erhebliches Maß an Irritation verbunden, was ebenfalls als Nachteil empfunden wird.

Bei einem *Duett^{™}* genannten Verfahren von Vascular Solutions, Minneapolis, MN, USA wird ein Ballon mit elliptischer Form in das Lumen eingebracht, der für zeitweiliges Verschließen der Punktionsstelle an der luminalen Seite sorgen soll, aber dennoch den Blutfluss durch das Gefäß nicht übermäßig behindern soll. Ferner wird eine die Koagulation unterstützende Mischung aus Kollagen und Thrombin appliziert. Dieses Verfahren ist insofern von Nachteil, als die Gefahr besteht, dass die injizierte Mischung unbeabsichtigt in das Blutgefäß gelangt, wobei durch den im Lumen angeordneten Ballon ferner die Gefahr besteht, dass der Durchfluss durch das Gefäß stark beeinträchtigt wird. Ein weiterer Nachteil besteht darin, dass der Ballon nach Verschluss der größeren Öffnung wieder herausgezogen werden muss, was eine weitere Öffnung hinterlässt, die jedoch einen deutlich geringeren Durchmesser hat.

Eine Vorrichtung zum Verschließen einer Öffnung in der Wand eines Hohlorgans, gemäß dem Oberbegriff des Anspruchs 1, ist aus der WO 99/38454 bekannt.

Alle insoweit beschriebenen Verfahren und Vorrichtungen für perkutanen Verschluss von Öffnungen in Wänden von Gefäßen oder Hohlorganen haben den Nachteil, dass sie entweder zu zeitaufwendig oder zu teuer sind, oder aber nicht ausreichend effizient genug, da sie den in dem zu verschließenden Blutgefäß herrschenden Druck von 100-160 mmHg nicht hinreichend entgegenwirken können.

Vor diesem Hintergrund liegt der vorliegenden Erfindung die Aufgabe zu Grunde, einen Verschlussstopfen der eingangs genannten Art bereitzustellen, mit dem ein punktiertes Gefäß oder Hohlorgan auf schnelle und einfache Weise verschlossen werden kann, so dass die Blutung sofort gestoppt wird.

Erfindungsgemäß wird diese Aufgabe gelöst durch einen Verschlussstopfen für eine Öffnung in einer Wand eines Gefäßes oder Hohlorgans eines tierischen oder menschlichen Körpers, insbesondere eines Blutgefäßes, mit einem in die Öffnung einsetzbaren und diese verschließenden Dichtkörper, und mit an dem Dichtkörper angeordneten Haltemitteln, die den Dichtkörper in der Wand verankern, wobei die Haltemittel Flügel umfassen, die nach Applikation des Verschlussstopfens in die Öffnung innen und außen an der Wand anliegen, wobei die sich nach Applikation des Verschlussstopfens in Längsrichtung des Gefäßes oder Hohlorgans befindlichen Flügel länger ausgebildet sind als die sich rechtwinklig zur Längsachse des Hohlorgans befindlichen Flügel, und wobei die sich rechtwinklig zur Längsachse des Hohlorgans befindlichen Flügel in ihrer aufgeprägten spannungsfreien Grundform derart bogenförmig erstrecken, dass diese nach Applikation des Verschlussstopfens in die Öffnung innen und außen an der Wand eines etwa hohlzylindrisch ausgebildeten Blutgefäßes anliegen, so dass der Stopfen an die Krümmung der hohlzylindrischen Form des Blutgefäßes oder Hohlorgans derart angepasst ist, dass eine Verletzung der Wand der letzteren vermieden wird.

Die der Erfindung zu Grunde liegende Aufgabe wird auf diese Weise vollkommen gelöst.

Der Verschlussstopfen wird mit dem Dichtkörper in der Öffnung platziert, so dass diese im Wesentlichen verschlossen ist und durch die Öffnung kein Blut mehr austreten kann. Um den Dichtkörper in der Öffnung zu halten, sind Haltemittel vorgesehen, die den Dichtkörper in oder an der Wand verankern.

Der Dichtkörper kann über ein bereits liegendes Schleusenröhrchen appliziert werden, wobei der Verschlussstopfen nach dem Austritt aus dem Schleusenröhrchen expandiert, ausklappt oder sich entspannt und auf diese Weise in oder an der Wand verankert.

Vor diesem Hintergrund wird ferner ein Operationskit offenbart, um eine Öffnung in einer Wand eines Gefäßes oder Hohlorgans eines tierischen oder menschlichen Körpers, insbesondere eines Blutgefäßes, zu verschließen, mit einer Hülse zum Einschieben in ein in die Öffnung eingesetztes Schleusenröhrchen, einem in der Hülse sitzenden Verschlussstopfen, und einem Ausschieber, um den Verschlussstopfen aus der Hülse auszuschieben.

Dabei ist es bevorzugt, wenn der Verschlussstopfen unter Vorspannung der Haltemittel in der Hülse klemmt und die Haltemittel sich beim Ausschieben des Verschlussstopfens entspannen und elastisch in ihre ausgeprägte, spannungsfreie Grundform zurückbewegen.

Vor diesem Hintergrund wird ferner eine Vorrichtung zum Applizieren eines derartigen Verschlussstopfens in einer Öffnung in einer Wand eines Gefäßes oder Hohlorgans eines tierischen oder menschlichen Körpers offenbart, insbesondere eines Blutgefäßes, mit einer Hülse zum Einschieben in ein in die Öffnung eingesetztes Schleusenröhrchen, wobei die Hülse zur Aufnahme eines Verschlussstopfens ausgebildet ist, und mit einem Ausschieber, um den Verschlussstopfen aus der Hülse auszuschieben. Die Hülse ist dabei vorzugsweise dazu ausgebildet, einen unter Vorspannung stehenden Verschlussstopfen aufzunehmen.

Während das Operationskit mit bereits vorbereitetem Verschlussstopfen geliefert wird, kann die neue Vorrichtung sozusagen vor Ort mit dem jeweils benötigten Verschlussstopfen "geladen" werden.

Die Erfindung basiert damit auf dem Gedanken, einen Verschlussstopfen zu verwenden, der sich selbst in der Öffnung verankert und bereits dadurch für einen Verschluss des Blutgefäßes sorgt. Auf diese Weise ist es nicht erforderlich, nach dem Applizieren des Verschlussstopfens noch über längere Zeit eine Restblutung durch manuelle Kompression oder entsprechende technische Vorkehrungen zu unterdrücken. Die Blutung wird vielmehr sofort gestoppt, wenn der Verschlussstopfen sich in der Öffnung verankert hat.

Ein Verfahren zum perkutanen Verschließen einer Punktion in einem Gefäß oder einem Hohlorgan sieht also so aus, dass ein Verschlussstopfen durch ein bereits gelegtes Schleusenröhrchen zu der Öffnung gebracht wird und sich dort in der Wand verankert, sobald das Schleusenröhrchen gezogen wurde und der Verschlussstopfen dadurch freikommt.

Der Verschlussstopfen ist damit sowohl für das Abdichten der Öffnung als auch für die Verankerung zuständig, aufwendige Manipulationen wie bspw. bei einem Naht-Automaten sind nicht mehr erforderlich.

Die Haltemittel umfassen Flügel, die nach Applikation des Verschlussstopfens in die Öffnung innen und außen an der Wand anliegen.

Hier ist von Vorteil, dass die sowohl innen als auch außen an der Wand des Gefäßes oder Hohlorgans anliegenden Flügel zum einen verhindern, dass der Verschlussstopfen aus der Öffnung herausgedrückt wird, zum anderen aber auch verhindern, dass er in das Lumen hineingezogen wird. Darüber hinaus sorgen die innen und außen an der Wand anliegenden Flügel neben dem Dichtkörper, der bereits einen großen Teil des Durchmessers der Öffnung verschließt, für ein sofortiges Stoppen der Blutung. Insbesondere diese Flügel ermöglichen es, dass der Verschlussstopfen durch ein liegendes Schleusenröhrchen appliziert wird, was im Umkehrschluss bedeutet, dass der Durchmesser des Dichtkörpers geringer ist als der Durchmesser der durch das Schleusenröhrchen hervorgerufenen Öffnung in der Wand. Zwar verkleinert sich die Öffnung in der Wand nach dem Ziehen des Schleusenröhrchens, es lässt sich jedoch nicht mit Sicherheit vorhersagen, ob sich die Öffnung bereits nach dem Ziehen so weit verkleinert, dass der Dichtkörper allein zum sofortigen und vollständigen Verschluss ausreicht. Die Flügel tragen somit zum sicheren Verschluss unmittelbar nach dem Applizieren des neuen Verschlussstopfens bei.

Dabei ist es bevorzugt, wenn der Dichtkörper eine obere und eine untere Dichtfläche aufweist, wobei an jeder Dichtfläche Flügel angeordnet sind, wobei vorzugsweise die Dichtflächen als Vieleck ausgebildet sind und an jeder Kante einer Dichtfläche ein Flügel angeordnet ist.

Zwar ist es prinzipiell möglich, den Dichtkörper als zylindrischen Körper auszubilden. Wenn die Dichtflächen und damit in der Regel auch der Dichtkörper jedoch als Vieleck ausgebildet sind, lassen sich die Flügel unmittelbar an den Kanten der Dichtflächen anbringen. Dies hat den Vorteil, dass die Flügel beim Applizieren des Verschlussstopfens durch das Schleusenröhrchen hoch- bzw. heruntergeklappt sein können und sich erst entfalten, wenn das Schleusenröhrchen gezogen wird. Dieses "Umklappen" ist geometrisch einfacher und spannungsfreier an einer geraden als an einer runden oder gebogenen Kante zu realisieren.

Dabei ist es bevorzugt, wenn die Flügel sich in ihrer aufgeprägten, spannungsfreien Grundform etwa quer zur Längsrichtung des Dichtkörpers erstrecken.

Hier ist von Vorteil, dass die Flügel nach dem Freikommen aus dem Schleusenröhrchen bzw. einer sie durch das Schleusenröhrchen transportierenden Hülse automatisch in ihre Grundform zurückgehen, es ist also nicht erforderlich, die Flügel aktiv, beispielsweise durch Führungsdrähte, Nahtmaterial etc. abzuklappen, sobald der Verschlussstopfen in der richtigen Position in der Öffnung sitzt. Diese Maßnahme trägt also dazu bei, dass der Verschlussstopfen auf einfache Weise appliziert werden kann, wobei er sich wegen der sich außen und innen an die Wand anlegenden Flügel auch sozusagen automatisch richtig in der Öffnung positioniert.

Weiter ist es bevorzugt, wenn die Flügel mit Verankerungsmitteln versehen sind, die sich an der Wand oder in umgebendem Gewebe verhaken, wobei die Verankerungsmittel vorzugsweise als Widerhaken oder Nasen an den Flügeln ausgebildet sind.

Auch diese Maßnahme sorgt für eine schnelle und gute Abdichtung der Öffnung. Durch die Verankerungsmittel wird der Dichtkörper so fest in der Öffnung gehalten, dass es nicht erforderlich ist, nach dem Applizieren des Verschlussstopfens noch über längere Zeit eine manuelle oder sonstige Kompression auszuüben. Mit anderen Worten, selbst bei einem Gefäß mit hohem Innendruck oder hoher Fließgeschwindigkeit verankert sich der Stopfen schnell und sicher in der Wand des Gefäßes oder Hohlorgans.

Allgemein ist es bevorzugt, wenn die Flügel über eine formelastische Verbindung an dem Dichtkörper befestigt sind, wobei die Flügel vorzugsweise aus einem formelastischen Material bestehen.

Diese Maßnahme ist konstruktiv und damit unter Kostengesichtspunkten von Vorteil, denn die Formelastizität, mit der die Flügel aus ihrer angeklappten in die aufgeklappte Position überführt werden, wird durch das Material der Flügel oder deren Verbindung zu dem Dichtkörper bereitgestellt. Auch auf diese Weise sind zusätzliche Manipulationen oder die Verwendung von Federn etc. nicht erforderlich.

Dabei ist es weiter bevorzugt, wenn der Dichtkörper und die Flügel einstückig aus einem formelastischen Material gefertigt sind.

Diese Maßnahme ist konstruktiv von Vorteil, der Verschlussstopfen kann beispielsweise in Spritzgusstechnik hergestellt werden, wobei die Flügel in der aufgeprägten spannungsfreien Grundform seitlich vom Dichtkörper abstehen. Die Flügel werden dann nach oben und unten umgeklappt, so dass der Dichtkörper unter Vorspannung der Haltemittel, also der Flügel, entweder unmittelbar in ein bestehendes Schleusenröhrchen oder aber in eine entsprechende Vorrichtung eingesetzt werden kann, über die er durch das Schleusenröhrchen hindurch zu der Öffnung transportiert wird.

Das Material ist dabei vorzugsweise ausgewählt aus der Gruppe: Elastomer, Material mit Formgedächtniseffekt, Nitinol, Polymer mit Formgedächtniseffekt.

Wenn Elastomere verwendet werden, so wird deren Kautschukelastisches Verhalten ausgenutzt, sich auch nach starker Verformung wieder in ihre ursprüngliche Form zurückbewegen zu können.

Wenn ein Material mit Formgedächtniseffekt verwendet wird, so wird die Fähigkeit dieser Materialien genutzt, nach plastischer Deformation die ursprüngliche Form wieder annehmen zu können, sobald sie über eine charakteristische Temperatur erwärmt wurden. Ein aus einem solchen Material gefertigter Gegenstand "erinnert" sich also an seine Ausgangsform, weshalb der genannte Effekt auch *Memory-Effekt* genannt wird. Nitinol ist eine Nickel-Titan-Legierung mit einem derartigen Formgedächtniseffekt.

Es sind jedoch auch Polymere bekannt, die einen Formgedächtniseffekt aufweisen. Von besonderem Vorteil ist dabei, dass das Material im Körper resorbierbar sein kann.

Resorbierbare Polymere mit Formgedächtniseffekt sind im Stand der Technik vielfach beschrieben; siehe beispielsweise US 6,160,084, US 2003/0055198 A1 oder EP 1 000 958 A1.

Sofern der Verschlussstopfen aus einem Formgedächtnismaterial besteht, kann er in seiner spannungsfreien Grundform so hergestellt werden, dass die Flügel von außen und von innen auf die Wand des Gefäßes oder Hohlorgans drücken. Der Verschlussstopfen wird dann erhitzt und in seine temporäre Form gebracht, in der er durch das Schleusenröhrchen hindurch appliziert werden kann. Diese temporäre Form wird durch Abkühlen unterhalb der so genannten Übergangstemperatur fixiert. Wird der Verschlussstopfen jetzt wieder erwärmt, stellt sich die ursprüngliche Form wieder ein. Es sind Polymersysteme verfügbar, bei denen die Übergangstemperatur im Bereich der Raum- oder Körpertemperatur liegt. Derartige Polymersysteme sind in den oben genannten Schutzrechten beschrieben und werden angeboten beispielsweise von der Firma mnemoScience GmbH, Pauwelsstraße 19, D-52074 Aachen, Deutschland.

Ein aus einem derartigen Polymersystem gefertigter Verschlussstopfen wird ggf. unter geringer Vorspannung in der Hülse des erfindungsgemäßen Operationskits gehalten, wobei der Verschlussstopfen sich in seiner temporären Form befindet und auf einer Temperatur deutlich unterhalb der Körpertemperatur gehalten wird. Nach dem Applizieren des Verschlussstopfens kommt dieser mit Körperflüssigkeit in Kontakt und erwärmt sich auf eine Temperatur oberhalb der Übergangstemperatur. Der Verschlussstopfen nimmt dann seine dauerhafte, spannungsfreie Grundform wieder ein, was bedeutet, dass die Flügel abklappen und den Dichtkörper in der Öffnung an der Wand verankern.

Auch bei einem Material mit Formgedächtnis kann das Ausklappen der Flügel natürlich überwiegend oder ausschließlich durch die Elastizität oder eine Klappbarkeit bewirkt werden.

Da das Material resorbierbar ist, wird es im Laufe der nächsten Tage und Wochen allmählich abgebaut, wobei die Gefäßwandzellen in die sich bei der Resorption ergebende Lücke automatisch hineinwachsen. Nach Beendigung des vollständigen Abbaus des Verschlussstopfens hat sich die Gefäßwand im Bereich der Punktionsstelle so weit neu gebildet, dass die Öffnung sicher verschlossen bleibt.

Dabei ist es bevorzugt, wenn das Material mit medizinisch wirksamen Substanzen beschichtet und/oder versetzt ist, beispielsweise mit Kollagen beschichtet ist und/oder ein Antibiotikum in das Material eingemischt ist.

Auf diese Weise kann zum einen der Heilungsprozess der Wand gefördert werden, wobei zum anderen beispielsweise durch das Antibiotikum das Auftreten von Entzündungen vermieden werden kann.

Allgemein ist es bevorzugt, wenn der Verschlussstopfen aus einem im Körper resorbierbaren Polymer mit Formgedächtniseffekt in Spritzgusstechnik hergestellt ist.

Bei dieser Maßnahme ist von Vorteil, dass der Verschlussstopfen preiswert herzustellen ist, wobei er aus den oben genannten Gründen schnell und einfach appliziert werden kann, so dass für einen sicheren und schnellen Verschluss der Öffnung in der Wand des Gefäßes oder Hohlorgans gesorgt wird.

Die Haltemittel des Verschlussstopfens, die sich nach der Applikation in Längsrichtung des Gefäßes oder Hohlorgans befinden, sind länger ausgebildet als die sich rechtwinklig zur Längsachse des Hohlorgans befindlichen Haltemittel. Die sich rechtwinklig zur Längsachse des Hohlorgans befindlichen Haltemittel erstrecken sich in ihrer aufgeprägten spannungsfreien Grundform derart bogenförmig, dass diese nach Applikation des Verschlussstopfens in die Öffnung innen und außen an der Wand eines etwa hohlzylindrisch ausgebildeten Blutgefäßes anliegen.

Diese Maßnahmen haben den Vorteil, dass der Stopfen an die Krümmung der hohlzylindrischen Form des Blutgefäßes oder Hohlorgans derart angepasst ist, dass eine Verletzung der Wand der Letzteren vermieden wird und gleichzeitig ein verschlussdichter Sitz des Stopfens sichergestellt wird.

Bei dem Operationskit kann der Ausschieber nach oben über einen an der Hülse vorgesehenen Anschlag so weit überstehen, dass er beim Einschieben in die Hülse den Verschlussstopfen gerade so weit nach unten aus der Hülse herausschiebt, dass die unteren Haltemittel aus der Hülse freikommen und sich entspannen.

Bei dieser Maßnahme ist von Vorteil, dass der Anschlag lediglich so weit wie möglich in die Hülse hineingeschoben werden muss, um den Verschlussstopfen zunächst teilweise aus der Hülse herauszuschieben. Es ist also nicht erforderlich, auf den genauen Einschubweg des Ausschiebers zu achten.

Da das proximale Ende der Hülse im Lumen des zu verschließenden Gefäßes oder Hohlorgans liegt, ragt der Verschlussstopfen jetzt mit seinem unteren Ende in das Lumen hinein, wo sich die Haltemittel, also insbesondere die Flügel, entspannen und eine Art Teller bilden. Wird jetzt das Schleusenröhrchen und damit die Hülse aus der Öffnung herausgezogen, so kommen die unteren Haltemittel in Anlage mit der Innenseite der Wand des Gefäßes oder Hohlorgans. Bei weiterem Herausziehen der Schleuse wird die Hülse dann von dem restlichen Verschlussstopfen heruntergezogen, so dass auch die oberen Haltemittel freikommen und sich entsprechend entspannen können.

Ein Verschlussstopfen wird also so in der in einer Wand vorgesehenen Öffnung platziert, dass er zunächst mit seinem unteren Ende in das Lumen des Blutgefäßes oder Hohlorgans gebracht wird, woraufhin das untere Ende zur Entfaltung der Haltemittel freigegeben wird. Beim Herausziehen der Schleuse und/oder der Hülse halten die unteren Haltemittel den Verschlussstopfen in der Öffnung fest, so dass dieser ganz aus dem Schleusenröhrchen bzw. der Hülse freikommt.

Dabei ist es insbesondere bevorzugt, wenn zwischen der Hülse und dem Ausschieber eine Arretierung vorgesehen ist.

Bei dieser Maßnahme ist von Vorteil, dass beim Einschieben der Hülse in das Schleusenröhrchen der Ausschieber nicht unbeabsichtigt betätigt wird, vielmehr muss zunächst die Arretierung entfernt oder überwunden werden. Als Arretierung kann beispielsweise eine überdrückbare Rastung, eine Feder oder aber auch ein Stopp verwendet werden, der zunächst entfernt werden muss.

Dabei ist es weiter bevorzugt, wenn außen an der Hülse ein Vorsprung vorgesehen ist, dessen Position an der Hülse derart auf die Länge des Schleusenröhrchens abgestimmt ist, dass die Hülse gerade so weit in das Schleusenröhrchen einschiebbar ist, dass sie an ihrem unteren Ende etwa bündig mit dem Schleusenröhrchen an dessen Austrittsöffnung abschließt.

Bei dieser Maßnahme ist von Vorteil, dass auch bei dem Einschieben der Hülse in das Schleusenröhrchen keine besondere Aufmerksamkeit darauf gerichtet werden muss, wie weit die Hülse eingeschoben werden kann. Damit ist es nicht erforderlich, eine Lagekontrolle für das proximale Ende der Hülse vorzunehmen.

Nachdem die erforderlichen Interventionen unter Verwendung des liegenden Schleusenröhrchens abgeschlossen sind, muss also lediglich die Hülse bis zum Anschlag in das Schleusenröhrchen eingeführt werden, um für die richtige Lage des Verschlussstopfens für dessen weitere Applikation zu sorgen. Danach wird der Ausschieber eingedrückt und nach kurzer Verweilzeit, innerhalb derer sich die unteren Haltemittel entfalten, das Schleusenröhrchen zusammen mit der darin befindlichen Hülse gezogen.

Auf diese Weise ist eine sehr einfache Applikation des Verschlussstopfens möglich, wobei das neue Verfahren insgesamt schnell und einfach durchzuführen ist, das Behandlungspersonal benötigt keine aufwendige Einweisung in das neue System.

Dabei ist es bevorzugt, wenn der Vorsprung ein außen auf der Hülse verschiebbar und arretierbar angeordneter Flansch ist. Hier ist von Vorteil, dass das Operationskit auf die Länge des jeweils verwendeten Schleusenröhrchens angepasst werden kann. Es ist damit nicht erforderlich, für Schleusenröhrchen unterschiedlicher Länge unterschiedliche Operationskits vorzusehen.

Andererseits ist es bevorzugt, wenn die Hülse einen Außendurchmesser aufweist, der auf Innendurchmesser von Schleusenröhrchen abgestimmt ist.

Hier ist von Vorteil, dass für jeweilige Schleusenröhrchen, deren unterschiedliche Durchmesser genormt sind, jeweils ein Operationskit vorgesehen werden kann, so dass ein Verschlussstopfen mit optimalem Durchmesser durch das jeweilige Schleusenröhrchen appliziert wird.

Offenbart wird außerdem ein Verfahren zum perkutanen Verschlieβen von Öffnungen in Wänden von Gefäßen oder Hohlorganen eines menschlichen oder tierischen Körpers, insbesondere von arteriellen oder venösen Punktionsstellen, mit den Schritten: (a) Einsetzen eines Schleusenröhrchens in die Öffnung; (b) Aufnehmen eines Verschlussstopfens in eine Hülse; (c) Einschieben der Hülse mit aufgenommenem Verschlussstopfen in das Schleusenröhrchen; (d) Ausschieben des Verschlussstopfens aus der in das Schleusenröhrchen eingeschobenen Hülse mittels eines Ausschiebers derart, dass der Verschlussstopfen in der Öffnung platziert wird, so dass diese im Wesentlichen verschlossen ist.

Weitere Vorteile ergeben sich aus der Beschreibung und der beigefügten Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele ohne die erfindungsgemäße bogenförmige flügel sind in der Zeichnung dargestellt und wird in der nachfolgenden Beschreibung näher erläutert. Es zeigen:
- Fig. 1: in einer perspektivischen Seitenansicht ein Blutgefäß mit gelegtem Schleusenröhrchen;
- Fig. 2: ein Operationskit aus Hülse, Ausschieber und Ver- schlussstopfen, das in das Schleusenröhrchen einge- führt wurde;
- Fig. 3: einen bei dem Operationskit aus Fig. 2 verwendbaren Verschlussstopfen in seiner temporären Form, beim Ausklappen der Haltemittel und in seiner spannungs- freien Grundform;
- Fig. 4: einen Flügel des Verschlussstopfens aus Fig. 3 mit angespritzter Nase;
- Fig. 5: einen Flügel des Verschlussstopfens aus Fig. 3 mit ausgestanzten Widerhaken; und
- Fig. 6: den Vorgang beim Applizieren des Verschlussstopfens unter Verwendung des Operationskits aus Fig. 2.

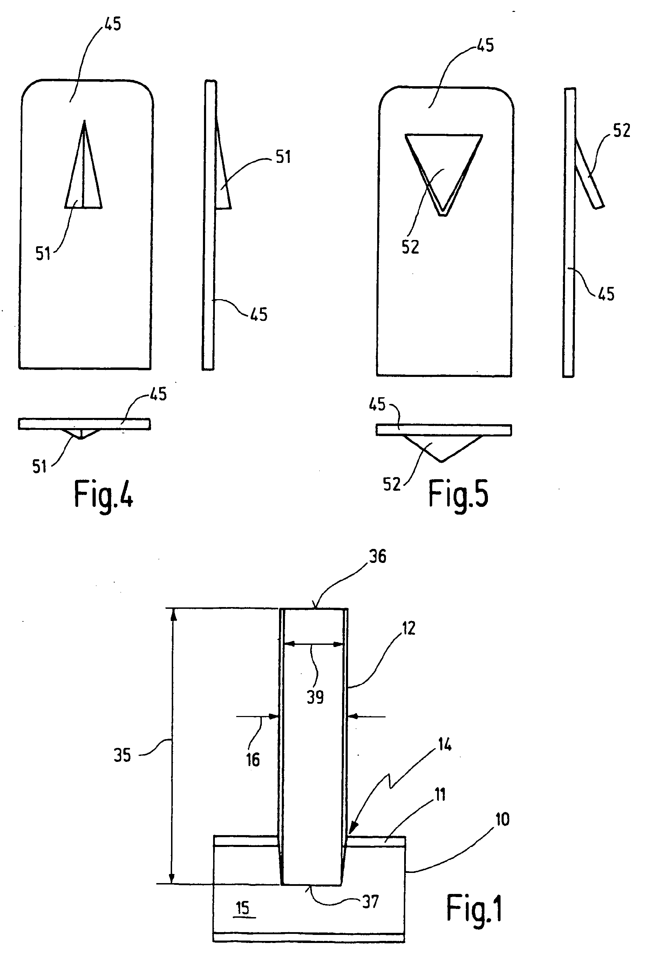

In Fig. 1 ist in einer schematischen Seitendarstellung und im Ausschnitt ein Blutgefäß 10 gezeigt, in dessen Wand 11 ein Schleusenröhrchen 12 eingeführt ist, so dass sich eine Öffnung 14 bildet, durch die das Lumen 15 des Blutgefäßes 10 zugänglich ist. Das Schleusenröhrchen 12 hat einen Außendurchmesser 16, der in etwa die Größe der Öffnung 14 bestimmt.

Ein derartiges Schleusenröhrchen 12 wird verwendet, um in das Lumen 15 einen Katheter einzuführen, wie er beispielsweise für radiologische oder kardiologische Interventionen benötigt wird.

Nach dem Ziehen des Schleusenröhrchens 12 verbleibt die Öffnung 14, was ohne weitere Behandlungsmaßnahmen zu fulminanten Blutungen insbesondere dann führen kann, wenn es sich bei dem Blutgefäß 10 beispielsweise um die Arteria femoralis handelt.

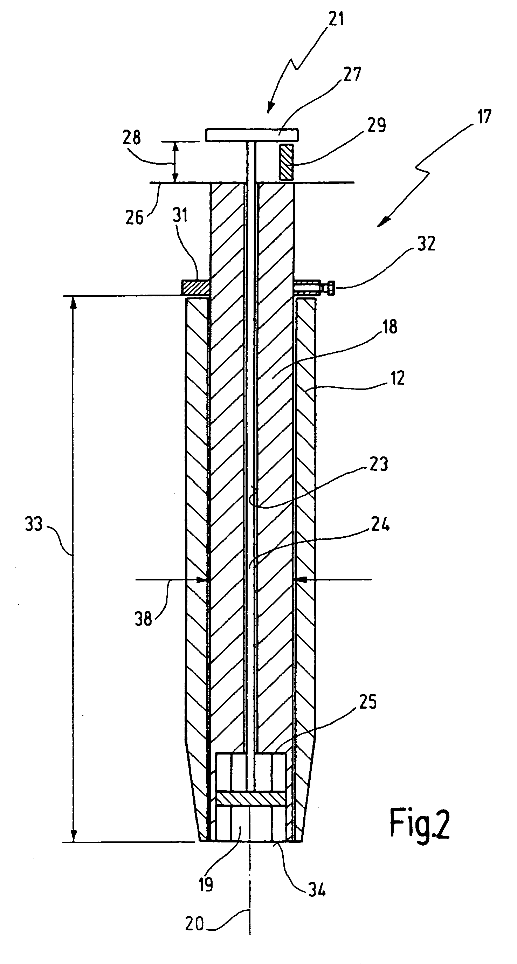

Um die Öffnung zu verschließen, wird ein Operationskit 17 verwendet, wie es im schematischen Schnitt und in Seitenansicht in der Fig. 2 dargestellt ist.

Das Operationskit 17 umfasst eine Hülse 18, in der ein Verschlussstopfen 19 sitzt, dessen bei 20 angedeutete Längsrichtung mit einem Ausschieber 21 zusammenfällt, der in einer in der Hülse 18 vorgesehenen Bohrung 23 mit seinem Stößel 24 geführt ist.

In der Hülse 18 ist eine Kammer 25 vorgesehen, in der der Verschlussstopfen 19 sitzt.

An ihrem distalen Ende ist die Hülse 18 mit einem Anschlag 26 versehen, der im einfachsten Fall ein quer angeordnetes Kunststoffblech sein kann. Der Stößel 24 trägt an seinem oberen Ende einen Kopf 27, der in der in Fig. 2 gezeigten Grundstellung des Operationskits 17 einen Abstand 28 zu dem Anschlag 26 aufweist. Dieser Abstand 28 ist deutlich kleiner als die Erstreckung des Verschlussstopfens 19 in seiner Längsrichtung 20, so dass beim vollständigen Einschieben des Stößels 24 in die Hülse 18 der Verschlussstopfen 19 nur etwa zur Hälfte aus der Kammer 25 herausgeschoben wird.

Damit der Ausschieber 21 nicht unbeabsichtigt betätigt wird, ist eine bei 29 angedeutete Arretierung vorgesehen, die es verhindert, dass der Kopf 27 auf den Anschlag 26 zu bewegt wird. Die Arretierung 29 kann ein Stopp sein, der zunächst entfernt werden muss, bevor der Ausschieber 21 betätigt werden kann. Es ist aber auch möglich, dass die Arretierung 29 eine überdrückbare Rastung oder eine Feder umfasst, so dass durch stärkeren Druck der Ausschieber 21 eingeschoben werden kann, was jedoch nicht unbeabsichtigt erfolgt.

Außen an der Hülse 18 ist ein ringförmiger Flansch 31 vorgesehen, der längs der Hülse verschiebbar ist und durch eine Rändelschraube 32 in seiner jeweiligen Position arretiert werden kann, so dass sich ein Abstand 33 zum unteren Ende 34 der Hülse 18 ergibt.

Der Flansch 31 wird so eingestellt, dass der Abstand 33 der Länge 35 des Schleusenröhrchens 12 entspricht; siehe Fig. 1.

Das in Fig. 2 gezeigte Operationskit 17 ist nun in das Schleusenröhrchen 12 eingeführt, wozu es in dessen Einführöffnung 36 (Fig. 1) so weit eingesteckt wird, bis der Flansch 31 an der Einführöffnung anschlägt. Das untere Ende 34 der Hülse 18 ist jetzt etwa bündig mit der Austrittsöffnung 37 des Schleusenröhrchens 12.

Damit das Operationskit 17 in das Schleusenröhrchen 12 eingeführt werden kann, ist es erforderlich, dass die Hülse 18 einen Außendurchmesser 38 aufweist, der geringfügig kleiner ist als ein in Fig. 1 bei 39 angedeuteter Innendurchmesser des Schleusenröhrchens 12.

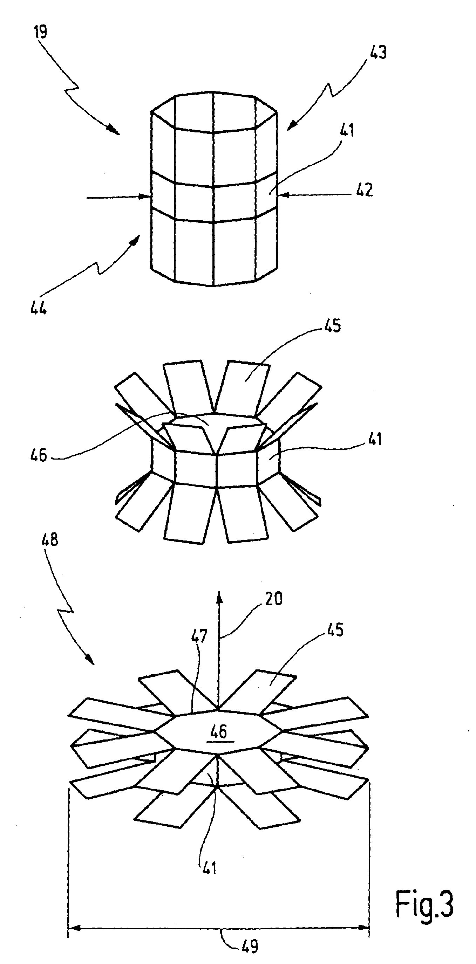

Der in Fig. 2 zu erkennende Verschlussstopfen ist in Fig. 3 detaillierter dargestellt.

Der Verschlussstopfen 19 umfasst zunächst einen Dichtkörper 41, dessen bei 42 angedeuteter Außendurchmesser derart ist, dass er in der Kammer 25 des Operationskits 17 platziert werden kann, wo er ggf. unter leichter Vorspannung gehalten wird.

An den Dichtkörper 41 schließen sich nach oben und unten obere bzw. untere Haltemittel 43 bzw. 44 an, die jeweils als Flügel 45 ausgebildet sind.

In der mittleren Darstellung in Fig. 3 sind die Flügel 45 zu einem gewissen Grad nach außen geneigt, so dass zwischen ihnen eine obere Dichtfläche 46 erkennbar ist. Diese obere Dichtfläche 46 sowie eine in der Fig. 3 nicht zu erkennende untere Dichtfläche sind jeweils als Vieleck ausgebildet, wobei an jeder Kante 47 eines Vielecks ein Flügel 45 angeordnet ist.

Der insoweit beschriebene Verschlussstopfen ist einstückig aus einem im Körper resorbierbaren Polymer mit Formgedächtniseffekt in Spritzgusstechnik hergestellt.

In Fig. 3 unten ist der Verschlussstopfen 19 in seiner spannungsfreien Grundform 48 dargestellt, in der sich die Flügel 45 etwa quer zur Längsrichtung 20 des Dichtkörpers 41 erstrecken. Die Flügel 45 weisen dabei eine bei 49 angedeutete Quererstreckung auf, die deutlich größer ist als der durch den Außendurchmesser 16 des Schleusenröhrchens 12 bedingte Durchmesser der Öffnung 14.

In Fig. 3 oben ist der Verschlussstopfen 19 in seiner temporären Form gezeigt, in der er unterhalb seiner Umwandlungstemperatur relativ spannungsfrei verbleibt. Wird die Temperatur des Verschlussstopfens 19 jedoch über die Umwandlungstemperatur erhöht, die oberhalb der Raumtemperatur, aber unterhalb der Körpertemperatur liegt, so klappen die Flügel 45 nach außen und gehen über die in Fig. 3 in der Mitte gezeigte Form wieder in die spannungsfreie Grundform über, die in Fig. 3 unten gezeigt ist. Dieses Ausklappen der Flügel 45 kann auch allein durch die Elastizität des Materials bewirkt werden.

Das Material des Verschlussstopfens 19 ist ein Polymersystem, wie es beispielsweise von der Firma mnemoScience geliefert wird. Der Verschlussstopfen 19 kann darüber hinaus mit einer geeigneten medizinischen Substanz, beispielsweise mit Kollagen, beschichtet sein, um den Heilungsprozess zu fördern. Ferner kann dem Polymer eine medizinisch wirksame Substanz, wie beispielsweise ein Antibiotikum, beigemischt werden, um Entzündungen zu verhindern.

Es sei noch erwähnt, dass das Material resorbierbar ist, so dass sich der Verschlussstopfen in an sich bekannter Weise allmählich im Körper auflöst, wobei durch den fortschreitenden Heilungsprozess der Dichtkörper wieder durch körpereigenes Gewebe ersetzt wird.

Wenn der Dichtkörper 41 in der Öffnung 14 appliziert ist, so verschließt er die Öffnung 14 nahezu vollständig. Da sein Auβendurchmesser 42 jedoch geringer ist als der Außendurchmesser 16 des Schleusenröhrchens 12, sorgen die Flügel 45 nicht nur für Halt des Dichtkörpers in der Öffnung 14, sie bilden vielmehr eine Art Teller oberhalb und unterhalb der Wand 11 und verschließen somit ggf. zwischen der Wand 11 und dem Dichtkörper 41 verbleibende Lücken.

Die Flügel 45 legen sich dabei dichtend und haltend sowohl außen als auch innen an die Wand 11 des Blutgefäßes 10 an, wobei diese Anlage ggf. unter leichter Vorspannung erfolgen kann. Auf diese Weise halten die Flügel 45 den Dichtkörper 41 in Position und sorgen für die zusätzlich erforderliche Abdichtung.

Um für einen besonders sicheren Halt des Verschlussstopfens 19 in der Öffnung 14 zu sorgen, sind die Flügel 45 mit Verankerungsmitteln versehen, die in den Fig. 4 und 5 dargestellt sind.

In Fig. 4 ist an dem Flügel 45 eine Nase 51 angespritzt, während in Fig. 5 aus dem Flügel 45 ein Widerhaken 52 ausgestanzt ist. Nase 51 bzw. Widerhaken 52 sorgen dafür, dass sich die ausgeklappten Flügel an der Wand 11 oder an umgebendem Gewebe verankern, so dass der Verschlussstopfen 19 fest in der Wand 11 sitzt. Die Geometrie der Flügel 45 kann auch antitraumatisch gerundet, sternförmig spitz oder anderweitig geeignet ausgelegt sein.

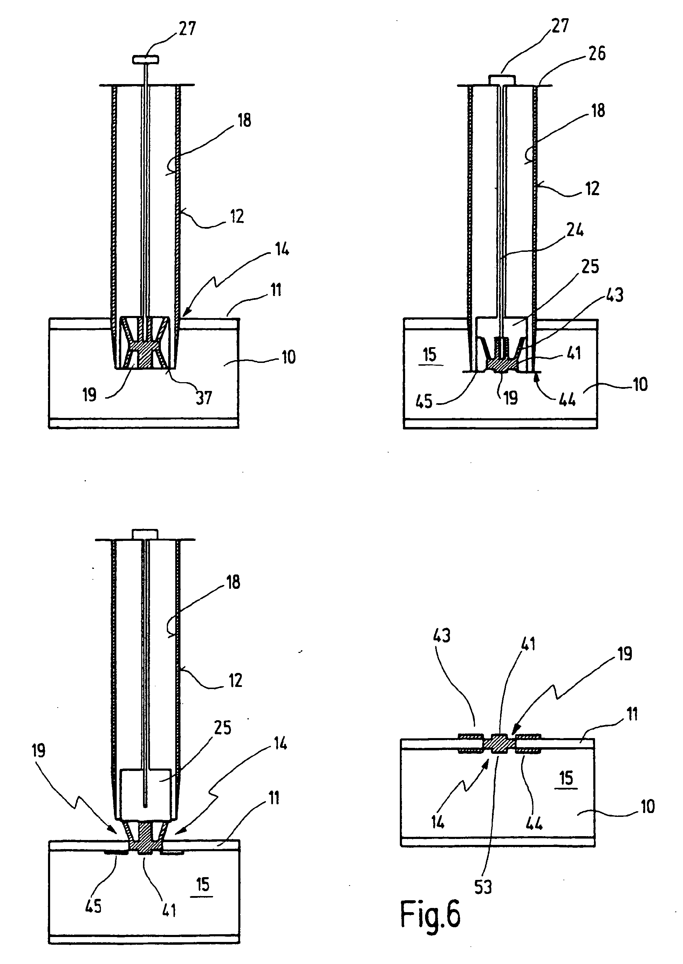

Wie die Applizierung des Verschlussstopfens 19 in der Öffnung 14 erfolgt, wird jetzt an Hand von Fig. 6 erörtert.

In Fig. 6 oben links ist der Zustand gezeigt, bei dem das Schleusenröhrchen 12 noch in der Öffnung 14 sitzt, in das Schleusenröhrchen 12 jedoch bereits die Hülse 18 eingefügt ist. Der Verschlussstopfen befindet sich jetzt unmittelbar an der Austrittsöffnung 37 des Schleusenröhrchens 12.

Jetzt wird der Kopf 27 nach unten auf den Anschlag 26 bewegt, wodurch der Stößel 24 den Verschlussstopfen 19 etwa halb aus der Kammer 25 heraus in das Lumen 15 des Blutgefäßes 10 hineindrückt. Die unteren Haltemittel 44, also die Flügel 45, können sich jetzt entspannen und in ihre spannungsfreie Grundform zurückgehen, in der sie sich etwa quer zum Dichtkörper 41 erstrecken und die Hülse 18 auf den Flügeln 45 aufsteht.

Dieser Übergang der Flügel 45 in die spannungsfreie Grundform erfolgt bspw. infolge der Elastizität des Materials oder weil der Verschlussstopfen 19 durch den Kontakt mit Körperflüssigkeit jetzt auf eine Temperatur oberhalb seiner Umwandlungstemperatur erwärmt wurde.

Wenn der Verschlussstopfen 19 nicht aus einem Material mit Formgedächtniseffekt sondern aus einem Elastomer gefertigt ist, so sitzt er unter stärkerer Vorspannung in der Kammer 25, die Flügel 45 gehen aber jetzt auf Grund ihrer Elastizität ebenfalls in ihre Ausgangsform zurück. Dieser Zustand ist in Fig. 6 oben rechts gezeigt.

Jetzt wird das Schleusenröhrchen 12 aus der Öffnung 14 herausgezogen, so dass der Dichtkörper 41 in der Öffnung 14 zu liegen kommt und sich die Flügel 45 der unteren Haltemittel 44 innen an die Wand 11 anlegen. Bereits jetzt ist die Öffnung 14 verschlossen, denn durch den Blutdruck in dem Lumen 15 wird der durch die Flügel 45 gebildete Teller nach außen gegen die Wand 11 gedrückt, wobei der Teller zusammen mit dem Verschlussstopfen 41, insbesondere dessen unterer Dichtfläche 53, die Öffnung 14 verschließt. Beim weiteren Herausziehen des Schleusenröhrchens 12 wird die Hülse 18 vollständig von dem Verschlussstopfen 19 abgezogen, so dass auch die oberen Haltemittel 43 aus der Kammer 25 freikommen und in ihre spannungsfreie Grundposition zurückgehen, wie dies oben bereits im Zusammenhang mit den unteren Haltemitteln 44 beschrieben wurde.

Das weitere Herausziehen des Schleusenröhrchens 12 ist in Fig. 6 unten links gezeigt, während in Fig. 6 unten rechts der in der Öffnung 14 verankerte Verschlussstopfen 19 gezeigt ist, der die Öffnung 14 nun vollständig verschließt. Die äußeren Haltemittel 43 drücken außen auf die Wand 11 und verhindern es so, dass der Verschlussstopfen 19 durch eine starke Strömung in dem Lumen 15 mitgerissen wird.

Ein Verschlussstopfen 19, wie er insoweit beschrieben wurde, wurde auf seine Eignung getestet, eine Punktionsstelle sicher zu verschließen. Der getestete Verschlussstopfen 19 wurde einstückig aus Nitinol gefertigt und hatte die in Fig. 3 gezeigte Form.

In ein Modell mit flüssigkeitsdurchströmten Schlauchmaterialien wurde ein 20 cm langes Stück Aorta vom Schwein eingefügt, wobei in dem Modell Innendrücke bis zu 200 mmHg getestet werden können. In die Aorta wurde dann der Verschlussstopfen 19 so eingefügt, dass er seine in Fig. 6 unten rechts gezeigte Form und Position einnimmt.

Es zeigte sich, dass der Verschlussstopfen 19 auch bei Innendrücken von 200 mmHg und großen Strömungsgeschwindigkeiten nicht nur sicher in der Öffnung 14 sitzt sondern darüber hinaus auch für eine vollständige Abdichtung der Öffnung 14 sorgt. Unmittelbar nach dem Applizieren des Verschlussstopfens 19 war die Öffnung 14 verschlossen und blieb dies auch während eines längeren Versuchslaufes.

## Patentansprüche

1. Verschlussstopfen für eine Öffnung (14) in einer Wand (11) eines Gefäßes oder Hohlorgans eines tierischen oder menschlichen Körpers, insbesondere eines Blutgefäßes (10), mit einem in die Öffnung (14) einsetzbaren und diese verschließenden Dichtkörper (41), und mit an dem Dichtkörper (41) angeordneten Haltemitteln (43, 44), die den Dichtkörper (41) in der Wand (11) verankern,
wobei die Haltemittel (43, 44) Flügel (45) umfassen, die nach Applikation des Verschlussstopfens (19) in die Öffnung (14) innen und außen an der Wand (11) anliegen,
wobei die sich nach Applikation des Verschlussstopfens in Längsrichtung des Gefäßes oder Hohlorgans befindlichen Flügel (45) länger ausgebildet sind als die sich rechtwinklig zur Längsachse des Hohlorgans befindlichen Flügel (45),
**dadurch gekennzeichnet, dass**
die sich rechtwinklig zur Längsachse des Hohlorgans befindlichen Flügel (45) in ihrer aufgeprägten spannungsfreien Grundform (48) derart bogenförmig erstrecken, dass diese nach Applikation des Verschlussstopfens (19) in die Öffnung (14) innen und außen an der Wand (11) eines etwa hohlzylindrisch ausgebildeten Blutgefäßes (10) anliegen, so dass der Stopfer an die Krümmung der hohlzylindrischen Form des Blutgefäßes oder Hohlorgans derart angepasst ist, dass eine Verletzung der Wand der letzteren vermieden wird.

2. Verschlussstopfen nach Anspruch 1, **dadurch gekennzeichnet, dass** der Dichtkörper (41) eine obere (46) und eine untere (53) Dichtfläche aufweist, wobei an jeder Dichtfläche (46, 53) Flügel (45) angeordnet sind.

3. Verschlussstopfen nach Anspruch 2, **dadurch gekennzeichnet, dass** die Dichtflächen (46, 53) als Vieleck ausgebildet sind und an jeder Kante (47) einer Dichtfläche (46, 53) ein Flügel (45) angeordnet ist.

4. Verschlussstopfen nach einem der Ansprüche 1 bis 3, **dadurch gekennzeichnet, dass** die Flügel (45) mit Verankerungsmitteln (51, 52) versehen sind, die sich an der Wand (11) oder in umgebendem Gewebe verhaken.

5. Verschlussstopfen nach Anspruch 4, **dadurch gekennzeichnet, dass** die Verankerungsmittel (51, 52) als Widerhaken (52) oder Nasen (51) an den Flügeln (45) ausgebildet sind.

6. Verschlussstopfen nach einem der Ansprüche 1 bis 5, **dadurch gekennzeichnet, dass** die Flügel (45) über eine formelastische Verbindung an dem Dichtkörper (41) befestigt sind.

7. Verschlussstopfen nach einem der Ansprüche 1 bis 6, **dadurch gekennzeichnet, dass** die Flügel (45) aus einem formelastischen Material bestehen.

8. Verschlussstopfen nach einem der Ansprüche 1 bis 7, **dadurch gekennzeichnet, dass** der Dichtkörper (41) und die Flügel (45) einstückig aus einem formelastischen Material gefertigt sind.

9. Verschlussstopfen nach Anspruch 7 oder 8, **dadurch gekennzeichnet, dass** das Material ausgewählt ist aus der Gruppe: Elastomer, Material mit Formgedächtniseffekt, Nitinol, Polymer mit Formgedächtniseffekt.

10. Verschlussstopfen nach einem der Ansprüche 7 bis 9, **dadurch gekennzeichnet, dass** das Material im Körper resorbierbar ist.

11. Verschlussstopfen nach einem der Ansprüche 7 bis 10, **dadurch gekennzeichnet, dass** das Material mit medizinisch wirksamen Substanzen beschichtet und/oder versetzt ist, bspw. mit Kollagen beschichtet ist und/oder ein Antibiotikum in das Material eingemischt ist.

12. Verschlussstopfen nach einem der Ansprüche 1 bis 11, **dadurch gekennzeichnet, dass** er einstückig aus einem im Körper resorbierbaren Polymer mit Formgedächtniseffekt in Spritzgusstechnik hergestellt ist.

## Claims

1. Sealing plug for an opening (14) in a wall (11) of a vessel or hollow organ of an animal or human body, in particular of a blood vessel (10), comprising a sealing body (41) insertable into the opening (14), which seals the same, and retaining means (43, 44) arranged on the sealing body (41), which anchor the sealing body (41) in the wall (11),
wherein the retaining means (43, 44) comprise blades (45), which fit against the inside and the outside of the wall (11) after the sealing plug (19) was applied into the opening (14),
wherein the blades (45) which extend along the longitudinal axis of the vessel or hollow organ, after the sealing plug was applied, are longer than the blades (45) which extent perpendicular to the longitudinal axis of the hollow organ,
**characterized in that**
the blades (45) which extend perpendicular to the longitudinal axis of the hollow organ are, in their tension free inherent basic form (48), arc-shaped, so that after the application of the sealing plug (19) into the opening (14) said blades fit against the inside and the outside of the wall (11) of an approximately cylindrically shaped vessel (10), so that the plug is adapted to the curvature of the hollow-cylindrical form of the blood vessel or hollow organ in such a manner that an injury of the wall of the latter is avoided.

2. Sealing plug of claim 1, **characterized in that** the sealing body (41) comprises an upper (46) and a lower (53) sealing surface, whereby blades (45) are arranged on each sealing surface (46, 53).

3. Sealing plug of claim 2, **characterized in that** the sealing surfaces (46, 53) are formed as a polygon and a blade (45) is arranged on each edge (47) of the sealing surface (46, 53).

4. Sealing means of any of claims 1 to 3, **characterized in that** the blades (45) are provided with anchoring means (51, 52) which clasp into the wall (11) or the surrounding tissue.

5. Sealing plug of claim 4, **characterized in that** the anchoring means (51, 52) on the blades (45) are shaped as barbed hooks (52) or projections (51).

6. Sealing plug of any of claims 1 to 5, **characterized in that** the blades (45) are attached to the sealing body (41) via a shape-elastic joint.

7. Sealing plug of any of claims 1 to 6, **characterized in that** the blades (45) consist of a shape-elastic material.

8. Sealing plug of any of claims 1 to 7, **characterized in that** the sealing body (41) and the blades (45) are integrally made of a shape-elastic material.

9. Sealing plug of claim 7 or 8, **characterized in that** the material is selected from the group: elastomer, material having a shape memory effect, nitinol, polymer having a shape memory effect.

10. Sealing plug of any of claims 7 to 9, **characterized in that** the material is absorbable in the body.

11. Sealing plug of any of claims 7 to 10, **characterized in that** the material is covered with medically effective substances and/or contains the same, for example the material is covered with collagen and/or an antibiotic is mixed into the material.

12. Sealing plug of any of claims 1 to 11, **characterized in that** it is integrally produced by injection molding technology of a polymer which is absorbable in the body and which comprises a shape memory effect.

## Revendications

1. Obturateur pour une ouverture (14) dans une paroi (11) d'un vaisseau ou d'un organe creux d'un corps animal ou humain, en particulier d'un vaisseau sanguin (10), comportant un corps étanche (41), destiné à être inséré dans l'ouverture (14) et obturant celle-ci, et comportant des moyens de fixation (43, 44), qui sont disposés sur le corps étanche (41) et par lesquels le corps étanche (41) est ancré dans la paroi (11),
lesdits moyens de retenue (43, 44) comportant des pattes (45) qui, après l'application de l'obturateur (19) dans l'ouverture (14), sont en appui à l'intérieur et à l'extérieur contre la paroi (11),
sachant que les pattes (45), qui sont situées dans la direction longitudinale du vaisseau ou de l'organe creux après l'application de l'obturateur, sont plus longues que les pattes (45) qui sont situées perpendiculairement à l'axe longitudinal de l'organe creux,
**caractérisé en ce que**
les pattes (45), orientées perpendiculairement à l'axe longitudinal de l'organe creux, s'étendent dans leur inhérent forme de base (48) sans tension, en forme de courbe, de telle sorte que lesdites pattes, après l'application de l'obturateur (19) dans l'ouverture (14), sont en appui à l'intérieur et à l'extérieur contre la paroi (11) d'un vaisseau sanguin (10) avec une forme sensiblement cylindrique creuse, de telle sorte que l'obturateur est ajusté à la forme cylindrique creuse du vaisseau sanguin ou de l'organe de manière à empêcher toute blessure de la paroi de ce dernier.

2. Obturateur selon la revendication 1, **caractérisé en ce que** le corps étanche (41) comporte une surface d'étanchéité supérieure (46) et une surface d'étanchéité inférieure (53), les pattes (45) étant agencées sur chaque surface d'étanchéité (46, 53).

3. Obturateur selon la revendication 2, **caractérisé en ce que** les surfaces d'étanchéité (46, 53) sont réalisées en forme de polygone et sur chaque arête (47) d'une surface d'étanchéité (46, 53) est agencée une patte (45).

4. Obturateur selon l'une quelconque des revendications 1 à 3, **caractérisé en ce que** les pattes (45) sont munies de moyens d'ancrage (51, 52) qui s'accrochent dans la paroi (11) ou dans le tissu environnant.

5. Obturateur selon la revendication 4, **caractérisé en ce que** les moyens d'ancrage (51, 52) sont réalisés sous la forme de barbes (52) ou de becs (51) sur les pattes (45).

6. Obturateur selon l'une quelconque des revendications 1 à 5, **caractérisé en ce que** les pattes (45) sont fixées contre le corps étanche (41) par l'intermédiaire d'un assemblage à élasticité de forme.

7. Obturateur selon l'une quelconque des revendications 1 à 6, **caractérisé en ce que** les pattes (45) sont réalisées dans un matériau à élasticité de forme.

8. Obturateur selon l'une quelconque des revendications 1 à 7, **caractérisé en ce que** le corps étanche (41) et les pattes (45) sont réalisés d'un seul tenant dans un matériau à élasticité de forme.

9. Obturateur selon la revendication 7 ou 8, **caractérisé en ce que** le matériau est choisi dans le groupe : élastomère, matériau à mémoire de forme, nitinol, polymère à mémoire de forme.

10. Obturateur selon l'une quelconque des revendications 7 à 9, **caractérisé en ce que** le matériau est résorbable dans le corps.

11. Obturateur selon l'une quelconque des revendications 7 à 10, **caractérisé en ce que** le matériau est revêtu de substances à effet médicinal et/ou est transplanté, par exemple revêtu de collagène et/ou un antibiotique est ajouté dans le matériau.

12. Obturateur selon l'une quelconque des revendications 1 à 11, **caractérisé en ce qu'**il est réalisé en une seule pièce par la technique de moulage par injection dans un polymère à mémoire de forme, résorbable dans le corps.
